# EUROPEAN PATENT SPECIFICATION

(11) **EP 2 003 209 B1**
(45) Date of publication and mention of the grant of the patent: **10.06.2020**
(21) Application number: 07737522.8
(22) Date of filing: 28.02.2007
(51) Int. Cl.: C12P 13/04, C12P 13/08, C12P 13/14, C12P 13/22

(54) **METHOD FOR PRODUCING L-AMINO ACID**
VERFAHREN ZUR HERSTELLUNG VON L-AMINOSÄURE
PROCEDE DE PRODUCTION D'ACIDE L-AMINE

(30) Priority: 03.03.2006 JP 2006057528
(43) Date of publication of application: 17.12.2008
(73) Proprietor: Ajinomoto Co., Inc., Tokyo 104-8315 (JP)
(72) Inventor: USUDA, Yoshihiro, Kawasaki-shi Kanagawa 210-8681 (JP); MATSUI, Kazuhiko, Kawasaki-shi Kanagawa 210-8681 (JP)
(74) Representative: Hoffmann Eitle
(86) International application number: PCT/JP2007/053803
(87) International publication number: WO 2007/100009

(56) References cited:
- EP-A1- 0 593 792
- EP-A1- 1 253 195
- EP-A1- 1 715 056
- EP-A2- 1 715 055
- WO-A1-2005/085419
- WO-A2-2005/073390
- JP-A- 2003 274 988
- JP-A- 2005 073 697
- US-A- 3 616 217
- BARBIRATO F ET AL: "1,3-PROPANEDIOL PRODUCTION BY FERMENTATION: AN INTERESTING WAY TO VALORIZE GLYCERIN FROM THE ESTER AND ETHANOL INDUSTRIES", INDUSTRIAL CROPS AND PRODUCTS, ELSEVIER, NL, vol. 7, no. 2/03, 1 January 1998 (1998-01-01), pages 281-289, XP000990421, ISSN: 0926-6690, DOI: 10.1016/S0926-6690(97)00059-9
- GONZALEZ-PAJUELO M. ET AL.: 'Production of 1,3-propanediol by clostridium butyricum VPI 3266 using a synthetic medium and raw glycerol' J. IND. MICROBIOL. BIOTECHNOL. vol. 31, no. 9, 2004, pages 442 - 446, XP003016874

### SEQUENCE LISTING

<110> Ajinomoto Co., Inc.
<120> Method for Producing L-Amino Acid
<130> C753-C7031
<150> JP2006-57528
   <151> 2006-03-03
<160> 4
<170> PatentIn version 3.3
<210> 1
   <211> 33
   <212> DNA
   <213> Artificial
<220>
   <223> primer 1
<400> 1
   ggaagatcta tttgccttcg cacatcaacc tgg 33
<210> 2
   <211> 30
   <212> DNA
   <213> Artificial
<220>
   <223> primer 2
<400> 2
   cggggtacct tgtaaatatt ttaacccgcc 30
<210> 3
   <211> 56
   <212> DNA
   <213> Artificial
<220>
   <223> primer 3
<400> 3
   ggaagatcta aggagacctt aaatgagcga cacaacgatc ctgcaaaaca gtaccc 56
<210> 4
   <211> 39
   <212> DNA
   <213> Artificial
<220>
   <223> primer 4
<400> 4
   cggggtacct cgtagaggtt tactggcgct tatccagcg 39

## Description

### Technical Field

The present invention relates to a method for producing an L-amino acid using a microorganism. L-amino acids are used in various fields such as for use in seasonings, food additives, feed additives, chemicals, and drugs.

### Background Art

L-amino acids such as L-threonine and L-lysine are industrially produced by fermentation using amino acid-producing bacteria such as *Escherichia* bacteria having an ability to produce these L-amino acids. As these amino acid-producing bacteria, bacterial strains isolated from nature, artificial mutants of those bacterial strains, recombinants of those bacterial strains in which L-amino acid biosynthetic enzymes are enhanced by gene recombination, or the like, are used. Examples of the methods for producing L-threonine include, for example, the methods described in Patent documents 1 to 4. Examples of the methods for producing L-lysine include, for example, the methods described in Patent documents 5 to 8.

In the industrial production of L-amino acids by fermentation, saccharides, i.e., glucose, fructose, sucrose, blackstrap molasses, starch hydrolysate, and so forth, are used as carbon sources.
[Patent document 1] Japanese Patent Laid-open (JP-A, Kokai) No. 5-304969
[Patent document 2] International Patent Publication WO98/04715
[Patent document 3] Japanese Patent Laid-open No. 05-227977
[Patent document 4] U.S. Patent Published Application No. 2002/0110876
[Patent document 5] Japanese Patent Laid-open No. 10-165180
[Patent document 6] Japanese Patent Laid-open No. 11-192088
[Patent document 7] Japanese Patent Laid-open No. 2000-253879
[Patent document 8] Japanese Patent Laid-open No. 2001-057896

WO2005/073390 teaches a method for producing L-amino acid comprising culturing a microorganism from the family Enterobacteriaceae using glycerol as carbon source, accumulating the L-amino acid and isolating it.

### Disclosure of the Invention

### Object to be Achieved by the Invention

The present invention intends to provide a method for producing an L-amino acid at a lower cost by using crude glycerol from biodiesel fuel production as a new raw material, as compared to conventional methods for producing L-amino acids by fermentation using microorganisms, which mainly utilize saccharides as the carbon source.

### Means for Achieving the Object

The inventors of the present invention assiduously conducted various research in order to achieve the foregoing object. As a result, they found that, by culturing a bacterium belonging to the family *Enterobacteriaceae* and having an L-amino acid-producing ability in a medium containing crude glycerol from biodiesel fuel production as the carbon source, L-amino acids could be produced in an equivalent or higher amount as compared to methods using saccharides as the carbon source. Furthermore, they also found that low purity crude glycerol, crude glycerol from biodiesel fuel production, produced as a by-product in the production of biodiesel fuel, which is industrially produced worldwide, had higher a growth promoting effect as glycerol, as compared to pure glycerol. The present invention was accomplished on the basis of these findings.

That is, the present invention provides the following.
(1) A method for producing an L-amino acid, which comprises culturing a bacterium belonging to the family *Enterobacteriaceae* and having an L-amino acid-producing ability in a medium containing crude glycerol from biodiesel fuel production as the carbon source to produce and accumulate the L-amino acid in culture, and collecting the L-amino acid from the culture.
(2) The method as described above, wherein the initial concentration of the glycerol in the medium is 1 to 30% w/v.
(3) The method as described above, wherein the bacterium belongs to the genus *Escherichia.*
(4) The method as described above, wherein the bacterium is belongs to the genus *Pantoea.*
(5) The method as described above, wherein the bacterium is *Escherichia coli.*
(6) The method as described above, wherein the L-amino acid is L-threonine or L-lysine.
(7) The method as described above, wherein the L-amino acid is L-threonine, and the activity or activities of one or more kinds of enzymes selected from the group consisting of aspartate semialdehyde dehydrogenase, aspartokinase I, homoserine kinase, aspartate aminotransferase, and threonine synthase encoded by the *thr* operon are enhanced in the bacterium.
(8) The method as described above, wherein the L-amino acid is L-lysine, and the activity or activities of one or more kinds of enzymes selected from the group consisting of dihydrodipicolinate reductase, diaminopimelate decarboxylase, diaminopimelate dehydrogenase, phosphoenolpyruvate carboxylase, aspartate aminotransferase, diaminopimelate epimerase, aspartate semialdehyde dehydrogenase, tetrahydrodipicolinate succinylase, and succinyl diaminopimelate deacylase are enhanced, and/or the activity of lysine decarboxylase is attenuated in the bacterium.
(9) The method as described above, wherein the L-amino acid is L-glutamic acid, and the activity or activities of one or more kinds of enzymes selected from the group consisting of glutamate dehydrogenase, citrate synthase, phosphoenolpyruvate carboxylase, and methyl citrate synthase are enhanced, and/or the activity of α-ketoglutarate dehydrogenase is attenuated in the bacterium.
(10) The method as described above, wherein the L-amino acid is L-tryptophan, and the activity or activities of one or more kinds of enzymes selected from the group consisting of phosphoglycerate dehydrogenase, 3-deoxy-D-arabinoheptulonate-7-phosphate synthase, 3-dehydroquinate synthase, shikimate dehydratase, shikimate kinase, 5-enolpyruvate shikimate 3-phosphate synthase, chorismate synthase, prephenate dehydratase, and chorismate mutase are enhanced in the bacterium.

### Best Mode for Carrying out the Invention

Hereafter, the present invention will be explained in detail.

### <1> Glycerol used in the present invention

Glycerol refers to a substance of the nomenclatural name of propane-1,2,3-triol. In the present invention, crude glycerol refers to industrially produced glycerol which contains impurities, and which is obtained from biodiesel fuel production. Crude glycerol is industrially produced by by the esterification reaction for biodiesel fuel production. Biodiesel fuel refers to aliphatic acid methyl esters produced from fats or oils, and methanol by a transesterification, and crude glycerol is produced as a by-product of this reaction (refer to Fukuda, H., Kondo, A., and Noda, H., 2001, J. Biosci. Bioeng., 92, 405-416). In the biodiesel fuel production process, the alkaline catalyst method is used for the transesterification in many cases, and acids are added for neutralization. Therefore, crude glycerol which has a purity of about 70 to 95% by weight containing water and impurities is produced. Crude glycerol produced in the biodiesel fuel production contains, in addition to water, residual methanol, alkali salts such as NaOH as a catalyst, and an acid such as K₂SO₄, which is used for neutralizing the alkali as impurities. Although it depends on the manufacturers and production methods, the content of such salts and methanol reaches several percents. The crude glycerol preferably contains ions of the alkali, and the acid used for the neutralization of the alkali, such as sodium ions, potassium ions, chloride ions, and sulfate ions in an amount of from 2 to 7%, preferably 3 to 6%, more preferably 4 to 5.8%, based on the weight of the crude glycerol. Although methanol may not be present as an impurity, it is preferably present in an amount of 0.01% or less.

The crude glycerol may further contain trace amounts of metals, organic acids, phosphorus, aliphatic acids, and so forth. Examples of the organic acids include formic acid, acetic acid, and so forth, and although they may not be present as impurities, they are preferably present in an amount of 0.01% or less. As the trace amounts of metals contained in the crude glycerol, trace metals required for growth of microorganisms are preferred, and examples include, magnesium, iron, calcium, manganese, copper, zinc, and so forth. Magnesium, iron and calcium are preferably present in an amount of from 0.00001 to 0.1%, preferably 0.0005 to 0.1%, more preferably 0.004 to 0.05%, still more preferably 0.007 to 0.01%, in terms of the total amount based on the weight of the crude glycerol. Manganese, copper and zinc are preferably present in an amount of from 0.000005 to 0.01%, preferably 0.000007 to 0.005%, more preferably 0.00001 to 0.001%, in terms of the total amount.

The purity of the crude glycerol may be 10% or higher, preferably 50% or higher, more preferably 70% or higher, particularly preferably 80% or higher, and as long as the amounts of the impurities are within the aforementioned range, the purity of the glycerol may be 90% or higher.

In the present invention, the crude glycerol is produced in the production of biodiesel fuel. Furthermore, crude glycerol used in the present invention enables production of more L-amino acids as compared to when using an equal weight of reagent glycerol, when it is used as the carbon source. To produce more L-amino acids as compared to reagent glycerol means to increase the amino acid production amount by 5% or more, preferably 10% or more, more preferably 20% or more, as compared to when reagent glycerol is used as the carbon source. The "reagent glycerol" means glycerol marketed as regent grade, or glycerol having a purity equivalent to the purity of glycerol marketed as regent grade, it preferably has a purity of 99% by weight or higher, and pure glycerol is particularly preferred. The "reagent glycerol of the same amount as crude glycerol" means reagent glycerol of the same weight of the crude glycerol except for water, when the crude glycerol contains water.

In the present invention, the crude glycerol may be used after dilution with a solvent such as water. In such a case, the aforementioned descriptions concerning the contents of glycerol and impurities are applied to the crude glycerol before the dilution. That is, as for crude glycerol containing a solvent such as water, when the solvent is eliminated so that the content of the solvent is 30% by weight or less, preferably 20% by weight or less, more preferably 10% by weight or less, if the contents of glycerol and the impurities of the crude glycerol are within the aforementioned ranges of the contents of glycerol and impurities, such crude glycerol corresponds to the "crude glycerol" used in the present invention.

### <2> Bacteria used in the present invention

In the present invention, a bacterium belonging to the family *Enterobacteriaceae* and having an L-amino acid-producing ability is used.

The family *Enterobacteriaceae* encompasses bacteria belonging to the genera of *Escherichia, Enterobacter, Erwinia, Klebsiella, Pantoea, Photorhabdus, Providencia, Salmonella, Serratia, Shigella, Morganella, Yersinia,* and so forth. In particular, bacteria classified into the family *Enterobacteriaceae* according to the taxonomy used by the NCBI (National Center for Biotechnology Information) database (http://www.ncbi.nlm.nih.gov/Taxonomy/Browser/wwwtax.cgi?id =91347) are preferred.

A "bacterium belonging to the genus *Escherichia"* means that the bacterium is classified into the genus *Escherichia* according to the classification known to a person skilled in the art of microbiology, although the bacterium is not particularly limited to these. Examples of the bacterium belonging to the genus *Escherichia* used in the present invention include, but are not limited to, *Escherichia coli (E. coli).*

The bacterium belonging to the genus *Escherichia* that can be used in the present invention is not particularly limited. However, examples include, for example, the bacteria of the groups described in the work of Neidhardt et al. (Neidhardt F.C. Ed., 1996, Escherichia coli and Salmonella: Cellular and Molecular Biology/Second Edition, pp.2477-2483, Table 1, American Society for Microbiology Press, Washington, D.C.). Specific examples include the *Escherichia coli* W3110 (ATCC 27325), *Escherichia coli* MG1655 (ATCC 47076) derived from the prototype wild-type strain K12 strain, and so forth.

These strains are available from, for example, the American Type Culture Collection (Address: P.O. Box 1549, Manassas, VA 20108, United States of America). That is, accession numbers are given to each of the strains, and the strains can be ordered by using these numbers. The accession numbers of the strains are listed in the catalogue of the American Type Culture Collection.

A bacterium belonging to the genus *Pantoea* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii,* or the like, based on the nucleotide sequence analysis of 16S rRNA, etc. (Int. J. Syst. Bacteriol., 43, 162-173 (1993)). In the present invention, bacteria belonging to the genus *Pantoea* encompass such bacteria re-classified into the genus *Pantoea* as described above.

In the bacterium used for the present invention, in order to enhance glycerol assimilability, expression of the *glpR* gene (EP 1715056) may be attenuated, or expression of the glycerol metabolism genes (EP 1715055 A) such as *glpA, glpB, glpC, glpD, glpE, glpF, glpG, glpK, glpQ, glpT, glpX, tpiA*, *gldA, dhaK, dhaL, dhaM, dhaR, fsa,* and *talC* genes may be enhanced.

In the present invention, a "bacterium having an L-amino acid-producing ability" means a bacterium which can produce and secrete an L-amino acid into a medium when it is cultured in the medium. It preferably means a bacterium which can cause accumulation of an objective L-amino acid in the medium in an amount not less than 0.5 g/L, more preferably not less than 1.0 g/L. The "L-amino acid" encompasses L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. L-Threonine, L-lysine and L-glutamic acid are especially preferred.

Hereinafter, methods for imparting an L-amino acid-producing ability to such bacteria as mentioned above, or methods for enhancing an L-amino acid-producing ability of such bacteria as mentioned above, are described.

To impart the ability to produce an L-amino acid, methods which have been conventionally employed in the breeding of the coryneform bacteria or bacteria of the genus *Escherichia* (see "Amino Acid Fermentation", Gakkai Shuppan Center (Ltd.), 1st Edition, published May 30, 1986, pp. 77-100) can be applied. Such methods include the acquisition of an auxotrophic mutant, an L-amino acid analogue-resistant strain, or a metabolic regulation mutant, or the construction of a recombinant strain having enhanced expression of an L-amino acid biosynthetic enzyme. Here, in the breeding of L-amino acid-producing bacteria, one or two or more properties, such as auxotrophic mutation, analogue resistance, or metabolic regulation mutation, may be imparted. The expression of L-amino acid biosynthetic enzyme(s) can be enhanced singly or in combinations of two or more. Furthermore, the technique of imparting properties such as an auxotrophic mutation, analogue resistance, or metabolic regulation mutation may be combined with the technique of enhancing the biosynthetic enzymes.

An auxotrophic mutant strain, L-amino acid analogue-resistant strain, or metabolic regulation mutant strain with the ability to produce an L-amino acid can be obtained by subjecting a parent strain or wild-type strain to a conventional mutatagenesis, such as exposure to X-rays or UV irradiation, or a treatment with a mutagen such as N-methyl-N'-nitro-N-nitrosoguanidine, etc., then selecting those which exhibit an autotrophy, analogue resistance, or metabolic regulation mutation and which also have the ability to produce an L-amino acid.

Moreover, impartation or enhancement of an L-amino acid-producing ability can also be attained by enhancing an enzymatic activity by gene recombination. An example of the method for enhancing an enzymatic activity includes, for example, a method of modifying a bacterium so that expression of the gene encoding an enzyme involved in biosynthesis of an L-amino acid is enhanced. As for the method for enhancing expression of the gene, enhancement can also be attained by introducing an amplification plasmid prepared by introducing a DNA fragment containing the gene into an appropriate plasmid, for example, a plasmid vector containing at least the gene responsible for replication and proliferation of the plasmid in a microorganism, increasing the copy number of the gene on the chromosome by conjugation, transfer or the like, or introducing a mutation into the promoter region of the gene (refer to International Patent Publication WO95/34672).

When an objective gene is introduced into the aforementioned amplification plasmid or chromosome, any promoter may be used to express the gene so long as it functions in coryneform bacteria. The promoter may be the promoter of the gene, or a modified promoter. The expression amount of the gene can also be controlled by suitably choosing a promoter that is potent in coryneform bacteria, or by making the -35 and -10 regions of the promoter closer to a consensus sequence. Such methods of enhancing expression of a gene as mentioned above are described in International Patent Publication WO00/18935, European Patent Publication No. 1010755, and so forth.

Methods for imparting an L-amino acid-producing ability to bacteria and bacteria imparted with L-amino acid-producing ability are exemplified below.

### L-Threonine-producing bacteria

Preferred examples of microorganisms having L-threonine-producing ability include bacteria in which one or two kinds of activities of L-threonine biosynthesis system enzymes are enhanced. Examples of L-threonine biosynthetic enzymes include aspartokinase III gene (*lysC*), aspartate semialdehyde dehydrogenase (*asd*), aspartokinase I (*thrA*), homoserine kinase (*thrB*), and threonine synthase (*thrC*), which are encoded by the threonine operon, and aspartate aminotransferase (aspartate transaminase) (*aspC*). Mentioned in the parentheses after the name of each enzyme is the name of the gene encoding each enzyme (the same shall apply to the following descriptions). Among these enzymes, aspartate semialdehyde dehydrogenase, aspartokinase I, homoserine kinase, aspartate aminotransferase, and threonine synthase are particularly preferred. The genes encoding the L-threonine biosynthetic enzymes may be introduced into an *Escherichia* bacterium in which threonine decomposition is decreased. An example of an *Escherichia* bacterium in which threonine decomposition is decreased is the TDH6 strain which is deficient in threonine dehydrogenase activity (JP 2001-346578 A).

The activities of the L-threonine biosynthetic enzymes are inhibited by the end-product, L-threonine. Therefore, L-threonine biosynthetic enzymes are preferably modified so they are desensitized to feedback inhibition by L-threonine when constructing L-threonine-producing strains. The aforementioned *thrA, thrB,* and *thrC* genes constitute a threonine operon, and the threonine operon forms an attenuator structure. The expression of the threonine operon is inhibited by isoleucine and threonine which are present in the culture medium, and is also suppressed by attenuation. Therefore, the threonine operon is preferably modified by removing the leader sequence or attenuator in the attenuation region (refer to Lynn, S.P., Burton, W.S., Donohue, T.J., Gould, R.M., Gumport, R.L, and Gardner, J.F., J. Mol. Biol. 194:59-69 (1987); WO02/26993; WO2005/049808).

The native promoter of the threonine operon is located upstream of the threonine operon, and it may be replaced with a non-native promoter (refer to WO98/04715), or a threonine operon may be constructed in which the expression of a threonine biosynthesis gene is controlled by the repressor and promoter of λ-phage (EP 0593792). Furthermore, in order to modify a bacterium to be desensitized to feedback inhibition by L-threonine, a strain resistant to α-amino-β-hydroxyisovaleric acid (AHV) may be selected.

It is preferable to increase the copy number of the threonine operon that is modified as described above so that it is desensitized to feedback inhibition by L-threonine in the host bacterium, or to increase expression of such a modified operon by ligating it to a potent promoter. Besides amplification using a plasmid, the copy number can also be increased by transferring the threonine operon to a genome using a transposon or Mu-phage.

Besides enhancing the L-threonine biosynthetic genes, it is also preferable to enhance expression of genes involved in the glycolytic pathway, TCA cycle, or respiratory chain, genes that regulate the expression of these genes, or genes involved in sugar uptake. Examples of genes that are effective for L-threonine production include the transhydrogenase gene (*pntAB,* EP 733712 B), phosphoenolpyruvate carboxylase gene (*pepC*, WO95/06114), phosphoenolpyruvate synthase gene (*pps,* EP 877090 B), and pyruvate carboxylase gene derived from coryneform bacterium or *Bacillus* bacterium (WO99/18228, EP 1092776 A).

It is also preferable to enhance expression of a gene that imparts to the host L-threonine resistance, L-homoserine resistance, or both. Examples of genes that impart these resistances include the *rhtA* gene (Res. Microbiol., 154:123-135 (2003)), *rhtB* gene (EP 0994190 A), *rhtC* gene (EP 1013765 A), *yfiK* gene, and *yeaS* gene (EP 1016710 A). As for the method for imparting L-threonine resistance to the host, the methods described in EP 0994190 A and WO90/04636 can be referred to.

Examples of the L-threonine-producing bacteria and parent strains for deriving them include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No. 5,631,157), *E. coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A), and so forth.

The strain TDH-6 is deficient in the *thrC* gene, as well as being sucrose-assimilative, and the *ilvA* gene thereof has a leaky mutation. This strain also has a mutation in the *rhtA* gene, which imparts resistance to high concentrations of threonine or homoserine. The strain B-3996 contains the plasmid pVIC40 which was obtained by inserting a *thrA*BC* operon which includes a mutant *thrA* gene into a RSF1010-derived vector. This mutant *thrA* gene encodes aspartokinase homoserine dehydrogenase I which is substantially desensitized to feedback inhibition by threonine. The strain B-3996 was deposited on November 19, 1987 in the All-Union Scientific Center of Antibiotics (Nagatinskaya Street 3-A, 117105 Moscow, Russia) under the accession number RIA 1867. The strain was also deposited at the Russian National Collection of Industrial Microorganisms (VKPM) (1 Dorozhny proezd., 1 Moscow 117545, Russia) on April 7, 1987 under the accession number VKPM B-3996.

*E. coli* VKPM B-5318 (EP 0593792 B) may also be used as an L-threonine-producing bacterium or a parent strain for deriving it. The strain B-5318 is prototrophic with regard to isoleucine, and a temperature-sensitive lambda-phage Cl repressor and PR promoter replace the regulatory region of the threonine operon in plasmid pVIC40. The strain VKPM B-5318 was deposited at the Russian National Collection of Industrial Microorganisms (VKPM) (1 Dorozhny proezd., 1 Moscow 117545, Russia) on May 3, 1990 under the accession number VKPM B-5318.

The thrA gene which encodes aspartokinase homoserine dehydrogenase I of *Escherichia coli* has been elucidated (nucleotide positions 337 to 2799, GenBank accession NC_000913.2, gi: 49175990). The thrA gene is located between the *thrL* and *thrB* genes on the chromosome of *E. coli* K-12. The *thrB* gene which encodes homoserine kinase of *Escherichia coli* has been elucidated (nucleotide positions 2801 to 3733, GenBank accession NC 000913.2, gi: 49175990). The *thrB* gene is located between the thrA and *thrC* genes on the chromosome of *E. coli* K-12. The *thrC* gene which encodes threonine synthase of *Escherichia coli* has been elucidated (nucleotide positions 3734 to 5020, GenBank accession NC 000913.2, gi: 49175990). The *thrC* gene is located between the *thrB* gene and the *yaaX* open reading frame on the chromosome of *E. coli* K-12. All three genes function as a single threonine operon. To enhance expression of the threonine operon, the attenuator region which affects the transcription is desirably removed from the operon (WO2005/049808, WO2003/097839).

A mutant thrA gene which codes for aspartokinase homoserine dehydrogenase I resistant to feedback inhibition by threonine, as well as the *thrB* and *thrC* genes can be obtained as one operon from the well-known plasmid pVIC40 which is present in the threonine-producing *E. coli* strain VKPM B-3996. The plasmid pVIC40 is described in detail in U.S. Patent No. 5,705,371.

The *rhtA* gene is present at 18 min on the *E. coli* chromosome close to the *glnHPQ* operon, which encodes components of the glutamine transport system. The rhtA gene is identical to ORF1 (*ybiF* gene, nucleotide positions 764 to 1651, GenBank accession number AAA218541, gi:440181) and is located between the *pexB* and *ompX* genes. The unit expressing a protein encoded by the ORP1 has been designated the *rhtA* gene (*rht:* resistance to homoserine and threonine). Also, it was revealed that the rhtA23 mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A) .

The *asd* gene of *E. coli* has already been elucidated (nucleotide positions 3572511 to 3571408, GenBank accession NC_0O0913.1, gi:16131307), and can be obtained by PCR (polymerase chain reaction; refer to White, T.J. et al., Trends Genet, 5, 185 (1989)) utilizing primers prepared based on the nucleotide sequence of the gene. The *asd* genes of other microorganisms can also be obtained in a similar manner.

Also, the *aspC* gene of *E. coli* has already been elucidated (nucleotide positions 983742 to 984932, GenBank accession NC 000913.1, gi:16128895), and can be obtained by PCR. The *aspC* genes of other microorganisms can also be obtained in a similar manner.

### L-Lysine-producing bacteria

Examples of L-lysine-producing bacteria belonging to the genus *Escherichia* include mutants having resistance to an L-lysine analogue. The L-lysine analogue inhibits the growth of bacteria belonging to the genus *Escherichia,* but this inhibition is fully or partially desensitized when L-lysine is present in a medium. Examples of the L-lysine analogue include, but are not limited to, oxalysine, lysine hydroxamate, S-(2-aminoethyl)-L-cysteine (AEC), γ-methyllysine, α-chlorocaprolactam, and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine-producing bacterium of *Escherichia coli.* This bacterial strain was bred by conferring AEC resistance to the strain W3110, which was derived from *Escherichia coli* K-12. The resulting strain was designated *Escherichia coli* AJ13069 and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of L-lysine-producing bacteria and parent strains for deriving L-lysine-producing bacteria also include strains in which expression of one or more genes encoding an L-lysine biosynthetic enzyme are enhanced. Examples of such enzymes include, but are not limited to, dihydrodipicolinate synthase (*dapA*), aspartokinase (*lysC*), dihydrodipicolinate reductase (*dapB*), diaminopimelate decarboxylase (*lysA*), diaminopimelate dehydrogenase (*ddh*) (U.S. Patent No. 6,040,160), phosphoenolpyrvate carboxylase (*ppc*), aspartate semialdehyde dehydrogenease (*asd*), diaminopimelate epimerase (*dapF*), tetrahydrodipicolinate succinylase (*dapD*), succinyl diaminopimelate deacylase (*dapE*), and aspartase (*aspA*) (EP 1253195 A). Among these enzymes, dihydrodipicolinate reductase, diaminopimelate decarboxylase, diaminopimelate dehydrogenase, phosphoenolpyrvate carboxylase, aspartate aminotransferase, diaminopimelate epimerase, aspartate semialdehyde dehydrogenease, tetrahydrodipicolinate succinylase, and succinyl diaminopimelate deacylase are especially preferred. In addition, the parent strains may have an increased level of expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (WO2005/073390), or combinations thereof.

Examples of L-lysine-producing bacteria and parent strains for deriving L-lysine-producing bacteria also include strains having decreased or no activity of an enzyme that catalyzes a reaction which generates a compound other than L-lysine via a pathway which branches off from the biosynthetic pathway of L-lysine. Examples of these enzymes that catalyze a reaction which generates a compound other than L-lysine via a pathway which branches off from the biosynthetic pathway of L-lysine include homoserine dehydrogenase, lysine decarboxylase (U.S. Patent No. 5,827,698), and the malic enzyme (WO2005/010175).

A preferred example of an L-lysine producing strain is *E. coli* WC196ΔcadAΔldc/pCABD2 (WO2006/078039). This strain was obtained by introducing the plasmid pCABD2, which is disclosed in U.S. Patent No. 6,040,160, into the strain WC196 which has disrupted *cadA* and *ldcC* genes encoding lysine decarboxylase. The plasmid pCABD2 contains a mutant *dapA* gene derived from *Escherichia coli* encoding a dihydrodipicolinate synthase (DDPS) which has a mutation which results in desensitization to the feedback inhibition by L-lysine, a mutant *lysC* gene derived from *Escherichia coli* encoding aspartokinase III which has a mutation which results in desensitization to the feedback inhibition by L-lysine, the *dapB* gene derived from *Escherichia coli* encoding dihydrodipicolinate reductase, and the *ddh* gene derived from *Brevibacterium lactofermentum* encoding diaminopimelate dehydrogenase.

### L-Cysteine-producing bacteria

Examples of L-cysteine-producing bacteria and parent strains for deriving L-cysteine-producing bacteria include, but are not limited to, strains belonging to the genus *Escherichia,* such as *E. coli* JM15 which is transformed with different *cysE* alleles encoding feedback-resistant serine acetyltransferases (U.S. Patent No. 6,218,168, Russian patent application 2003121601); *E. coli* W3110 having over-expressed genes which encode proteins suitable for secreting substances toxic for cells (U.S. Patent No. 5,972,663); *E. coli* strains having lowered cysteine desulfohydrase activity (JP 11155571 A2); *E. coli* W3110 with increased activity of a positive transcriptional regulator for cysteine regulon encoded by the *cysB* gene (WO01/27307A1), and so forth.

### L-Leucine-producing bacteria

Examples of L-leucine-producing bacteria and parent strains for deriving L-leucine-producing bacteria include, but are not limited to, strains belonging to the genus *Escherichia,* such as *E. coli* strains resistant to leucine (for example, the strain 57 (VKPM B-7386, U.S. Patent No. 6,124,121)) or leucine analogues including β-2-thienylalanine, 3-hydroxyleucine, 4-azaleucine, 5,5,5-trifluoroleucine and so forth (JP 62-34397 B and JP 8-70879 A) ; *E. coli* strains obtained by the gene engineering method described in WO96/06926; *E. coli* H-9068 (JP 8-70879 A), and so forth.

The bacterium used for the present invention may be improved by enhancing expression of one or more genes involved in L-leucine biosynthesis. Examples include genes of the *leuABCD* operon, which preferably include a mutant *leuA* gene encoding isopropyl malate synthase desensitized to the feedback inhibition by L-leucine (US Patent 6,403,342). In addition, the bacterium used for the present invention may be improved by enhancing expression of one or more genes encoding proteins which excrete L-amino acid from the bacterial cell. Examples of such genes include the *b2682* and *b2683* genes (*ygaZH* genes) (EP 1239041 A2) .

### L-Histidine-producing bacteria

Examples of L-histidine-producing bacteria and parent strains for deriving L-histidine-producing bacteria include, but are not limited to, strains belonging to the genus *Escherichia,* such as *E. coli* strain 24 (VKPM B-5945, RU2003677), *E. coli* strain 80 (VKPM B-7270, RU2119536), *E. coli* NRRL B-12116 - B 12121 (U.S. Patent No. 4,388,405), *E. coli* H-9342 (FERM BP-6675) and H-9343 (FERM BP-6676) (U.S. Patent No. 6,344,347), *E. coli* H-9341 (FERM BP-6674) (EP 1085087), *E. coli* AI80/pFM201 (U.S. Patent No. 6,258,554), and so forth.

Examples of L-histidine-producing bacteria and parent strains for deriving L-histidine-producing bacteria also include strains in which expression of one or more genes encoding an L-histidine biosynthetic enzyme are enhanced. Examples of such genes include ATP phosphoribosyltransferase gene (*hisG*), phosphoribosyl AMP cyclohydrolase gene (*hisI*), phosphoribosyl-ATP pyrophosphohydrolase gene (*hisIE*), phosphoribosylformimino-5-aminoimidazole carboxamide ribotide isomerase gene (*hisA*), amidotransferase gene (*hisH*), histidinol phosphate aminotransferase gene (*hisC*), histidinol phosphatase gene (*hisB*), histidinol dehydrogenase gene (*hisD*), and so forth.

It is known that the L-histidine biosynthetic enzymes encoded by *hisG* and *hisBHAFI* are inhibited by L-histidine, and therefore the L-histidine-producing ability can be efficiently enhanced by introducing a mutation which confers resistance to this feedback inhibition into the gene encoding ATP phosphoribosyltransferase (*hisG*) (Russian Patent Nos. 2003677 and 2119536).

Specific examples of strains having an L-histidine-producing ability include *E. coli* FERM-P 5038 and 5048, which have been introduced with a vector carrying a DNA encoding an L-histidine-biosynthetic enzyme (JP 56-005099 A), *E. coli* strains introduced with a gene for an amino acid-export (EP 1016710 A), *E. coli* 80 strain imparted with sulfaguanidine, DL-1,2,4-triazole-3-alanine, and streptomycin-resistance (VKPM B-7270, Russian Patent No. 2119536), and so forth.

### L-Glutamic acid-producing bacteria

Examples of L-glutamic acid-producing bacteria and parent strains for deriving L-glutamic acid-producing bacteria include, but are not limited to, strains belonging to the genus *Escherichia,* such as *E. coli* VL334thrC⁺ (EP 1172433). *E. coli* VL334 (VKPM B-1641) is an L-isoleucine and L-threonine auxotrophic strain having mutations in *thrC* and *ilvA* genes (U.S. Patent No. 4,278,765). A wild-type allele of the *thrC* gene was transferred by the method of general transduction using a bacteriophage P1 grown on the wild-type *E. coli* strain K12 (VKPM B-7) cells. As a result, the L-isoleucine auxotrophic strain VL334thrC⁺ (VKPM B-8961), which is able to produce L-glutamic acid, was obtained.

Examples of L-glutamic acid-producing bacteria and parent strains for deriving L-glutamic acid-producing bacteria include, but are not limited to, strains in which expression of one or more genes encoding an L-glutamic acid biosynthetic enzyme is enhanced. Examples of such genes include genes encoding glutamate dehydrogenase (*gdhA*), glutamine synthetase (*glnA*), glutamate synthetase (*gltAB*), isocitrate dehydrogenase (*icdA*), aconitate hydratase (*acnA, acnB*), citrate synthase (*gltA*), methyl citrate synthase gene (*prpC*), phosphoenolpyruvate carboxylase (*ppc*), pyruvate dehydrogenase (*aceEF, lpdA*), pyruvate kinase (*pykA, pykF*), phosphoenolpyruvate synthase (*ppsA*), enolase (*eno*), phosphoglyceromutase (*pgmA, pgmI*), phosphoglycerate kinase (*pgk*), glyceraldehyde-3-phophate dehydrogenase (*gapA*), triose phosphate isomerase (*tpiA*), fructose bisphosphate aldolase (*fbp*), phosphofructokinase (*pfkA, pfkB*), glucose phosphate isomerase (*pgi*), and so forth. Among these enzymes, glutamate dehydrogenase, citrate synthase, phosphoenolpyruvate carboxylase, and methyl citrate synthase are preferred.

Examples of strains modified so that expression of the citrate synthetase gene, the phosphoenolpyruvate carboxylase gene, and/or the glutamate dehydrogenase gene are enhanced include those disclosed in EP 1078989 A, EP 955368 A, and EP 952221 A.

Examples of L-glutamic acid-producing bacteria and parent strains for deriving L-glutamic acid-producing bacteria also include strains with decreased or no activity of an enzyme that catalyzes synthesis of a compound other than L-glutamic acid via a pathway that branches off from the L-glutamic acid biosynthesis pathway. Examples of such enzymes include isocitrate lyase (*aceA*), α-ketoglutarate dehydrogenase (*sucA*), phosphotransacetylase (*pta*), acetate kinase (*ack*), acetohydroxy acid synthase (*ilvG*), acetolactate synthase (*ilvI*), formate acetyltransferase (*pfl*), lactate dehydrogenase (*ldh*), glutamate decarboxylase (*gadAB*), and so forth. Bacteria belonging to the genus *Escherichia* deficient in α-ketoglutarate dehydrogenase activity or having reduced α-ketoglutarate dehydrogenase activity and methods for obtaining them are described in U.S. Patent Nos. 5,378,616 and 5,573,945.

Specifically, these strains include the following:
*E. coli* W3110sucA::Km^{r}
*E. coli* AJ12624 (FERM BP-3853)
*E. coli* AJ12628 (FERM BP-3854)
*E. coli* AJ12949 (FERM BP-4881)

*E. coli* W3110sucA: :Km^{r} is a strain obtained by disrupting the α-ketoglutarate dehydrogenase gene (hereinafter also referred to as the *"sucA* gene") of *E. coli* W3110. This strain is completely deficient in α-ketoglutarate dehydrogenase.

Other examples of L-glutamic acid-producing bacterium include those which belong to the genus *Escherichia* and are resistant to an aspartic acid antimetabolite. These strains can also be deficient in α-ketoglutarate dehydrogenase activity and include, for example, *E. coli* AJ13199 (FERM BP-5807) (U.S. Patent No. 5,908,768), FERM P-12379, which additionally has a low L-glutamic acid-decomposing ability (U.S. Patent No. 5,393,671); AJ13138 (FERM BP-5565) (U.S. Patent No. 6,110,714), and so forth.

An example of an L-glutamic acid producing strain of *Pantoea ananatis* is, but is not limited to, the *Pantoea ananatis* AJ13355 strain. This strain was isolated from soil in Iwata-shi, Shizuoka-ken, Japan as a strain that can proliferate in a medium containing L-glutamic acid and a carbon source at a low pH. The *Pantoea ananatis* AJ13355 strain was deposited at the National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on February 19, 1998 and received an accession number of FERM P-16644. It was then converted to an international deposit under the provisions of Budapest Treaty on January 11, 1999. and received an accession number of FERM BP-6614. This strain was identified as *Enterobacter agglomerans* when it was isolated and deposited as the *Enterobacter agglomerans* AJ13355 strain. However, it was recently re-classified as *Pantoea ananatis* on the basis of the nucleotide sequencing of 16S rRNA and so forth.

Furthermore, examples of L-glutamic acid producing *Pantoea ananatis* strains include bacteria belonging to the genus *Pantoea* in which α-ketoglutarate dehydrogenase (αKGDH) activity is eliminated or reduced. Examples of such a strain include the AJ13356 strain (U.S. Patent No. 6,331,419) which corresponds to the AJ13355 strain in which the αKGDH-E1 subunit gene (*sucA*) is eliminated, and the SC17sucA strain (U.S. Patent No. 6,596,517), which is a *sucA* gene-deficient strain derived from the SC17 strain selected as a low phlegm production mutant strain. The AJ13356 strain was deposited at the National Institute of Bioscience and Human-Technology, Agency of Industrial Science and Technology, Ministry of International Trade and Industry (currently, the independent administrative agency, National Institute of Advanced Industrial Science and Technology, International Patent Organism Depositary (Tsukuba Central 6, 1-1, Higashi 1-Chome, Tsukuba-shi, Ibaraki-ken, Japan, postal code: 305-8566)) on February 19, 1998, and assigned an accession number of FERM P-16645. Then, the deposit was converted into an international deposit under the provisions of the Budapest Treaty on January 11, 1999, and assigned an accession number of FERM BP-6616. Although the AJ13355 and AJ13356 strains were deposited at the aforementioned depository as *Enterobacter agglomerans,* they are referred to as *Pantoea ananatis* in this specification. The SC17sucA strain was assigned a private number of AJ417, and deposited at the National Institute of Advanced Industrial Science and Technology, International Patent Organism Depositary on February 26, 2004, under an accession number of FERM BP-08646.

Examples of L-glutamic acid-producing *Pantoea ananatis* strain further include the SC17sucA/RSFCPG+pSTVCB strain, AJ13601 strain, NP106 strain, and NA1 strain. The SC17sucA/RSFCPG+pSTVCB strain is obtained by introducing the plasmid RSFCPG containing the citrate synthase gene (*gltA*), phosphoenolpyruvate carboxylase gene (*ppsA*), and glutamate dehydrogenase gene (*gdhA*) derived from *Escherichia coli,* and the plasmid pSTVCB containing the citrate synthase gene (*gltA*) derived from *Brevibacterium lactofermentum,* into the SC17sucA strain. The AJ13601 strain is selected from the SC17sucA/RSFCPG+pSTVCB strain as a strain showing resistance to L-glutamic acid of a high concentration at a low pH. Furthermore, the NP106 strain corresponds to the AJ13601 strain in which plasmid RSFCPG+pSTVCB is eliminated as described in the examples section. The AJ13601 strain was deposited at the National Institute of Advanced Industrial Science and Technology, International Patent Organism Depositary on August 18, 1999, and assigned an accession number FERM P-17516. Then, the deposit was converted to an international deposit under the provisions of the Budapest Treaty on July 6, 2000, and assigned an accession number FERM BP-7207.

### L-Phenylalanine-producing bacteria

Examples of L-phenylalanine-producing bacteria and parent strains for deriving L-phenylalanine-producing bacteria include, but are not limited to, strains belonging to the genus *Escherichia,* such as *E. coli* AJ12739 (tyrA::Tn10, tyrR) (VKPM B-8197), which is deficient in chorismate mutase, prephenate dehydrogenase, and the tyrosine repressor (WO03/044191); *E. coli* HW1089 (ATCC 55371) which contains a mutant *pheA34* gene encoding chorismate mutase and prephenate dehydratase desensitized to feedback inhibition (U.S. Patent No. 5,354,672); *E. coli* MWEC101-b (KR8903681); *E. coli* NRRL B-12141, NRRL B-12145, NRRL B-12146, and NRRL B-12147 (U.S. Patent No. 4,407,952). Also, as a parent strain, *E. coli* K-12 [W3110(tyrA)/pPHAB (FERM BP-3566) having genes encoding chorismate mutase and prephenate dehydratase desensitized to feedback inhibition, *E. coli* K-12 [W3110(tyrA)/pPHAD] (FERM BP-12659), *E. coli* K-12 [W3110(tyrA)/pPHATerm] (FERM BP-12662) and *E. coli* K-12 [W3110(tyrA)/pBR-aroG4, pACMAB] named as AJ12604 (FERM BP-3579) may be used (EP 488424 B1). Furthermore, L-phenylalanine-producing bacteria belonging to the genus *Escherichia* with an enhanced activity of the protein encoded by the *yedA* gene or the *yddG* gene may also be used (U.S. Patent Published Applications Nos. 2003/0148473 A1 and 2003/0157667 A1, WO03/044192).

### L-Tryptophan-producing bacteria

Examples of L-tryptophan-producing bacteria and parent strains for deriving L-tryptophan-producing bacteria include, but are not limited to, strains belonging to the genus *Escherichia,* such as *E. coli* JP4735/pMU3028 (DSM10122) and JP6015/pMU91 (DSM10123), which is deficient in the tryptophanyl-tRNA synthetase encoded by mutant *trpS* gene (U.S. Patent No. 5,756,345); *E. coli* SV164 (pGH5) having a *serA* allele encoding phosphoglycerate dehydrogenase free from feedback inhibition by serine and a *trpE* allele encoding anthranilate synthase free from feedback inhibition by tryptophan (U.S. Patent No. 6,180,373); *E. coli* AGX17 (pGX44) (NRRL B-12263) and AGX6(pGX50)aroP (NRRL B-12264) deficient in tryptophanase (U.S. Patent No. 4,371,614); *E. coli* AGX17/pGX50,pACKG4-pps in which phosphoenolpyruvate-producing ability is enhanced (WO9708333, U.S. Patent No. 6,319,696), and so forth. L-Typtophan-producing bacteria belonging to the genus. *Escherichia* with an enhanced activity of the protein encoded by the *yedA* gene or the *yddG* gene may also be used (U.S. Patent Published Application Nos. 2003/0148473 A1 and 2003/0157667 A1).

Examples of L-tryptophan-producing bacteria and parent strains for deriving L-tryptophan-producing bacteria also include strains in which one or more activities are enhanced of the following enzymes: anthranilate synthase (*trpE*), phosphoglycerate dehydrogenase (*serA*), 3-deoxy-D-arabinoheptulosonate-7-phosphate synthase (*aroG*), 3-dehydroquinate synthase (*aroB*), shikimate dehydrogenase (*aroE*), shikimate kinase (*aroL*), 5-enolpyruvylshikimate-3-phosphate synthase (*aroA*), chorismate synthase (*aroC*), prephenate dehydratase, chorismate mutase, and tryptophan synthase (*trpAB*). Prephenate dehydratase and chorismate mutase are encoded by the pheA gene as a bifunctional enzyme (CM-PD). Among these enzymes, phosphoglycerate dehydrogenase, 3-deoxy-D-arabinoheptulosonate-7-phosphate synthase, 3-dehydroquinate synthase, shikimate dehydratase, shikimate kinase, 5-enolpyruvylshikimate-3-phosphate synthase, chorismate synthase, prephenate dehydratase, chorismate mutase-prephenate dehydratase are especially preferred. The anthranilate synthase and phosphoglycerate dehydrogenase both suffer from feedback inhibition by L-tryptophan and L-serine, and therefore a mutation desensitizing the feedback inhibition may be introduced into these enzymes. Specific examples of strains having such a mutation include *E. coli* SV164 which harbors desensitized anthranilate synthase and a transformant strain obtained by introducing into the *E. coli* SV164 the plasmid pGH5 (WO94/08031), which contains a mutant serA gene encoding feedback-desensitized phosphoglycerate dehydrogenase.

Examples of L-tryptophan-producing bacteria and parent strains for deriving L-tryptophan-producing bacteria also include strains into which the tryptophan operon which contains a gene encoding inhibition-desensitized anthranilate synthase has been introduced (JP 57-71397 A, JP 62-244382 A, U.S. Patent No. 4,371,614). Moreover, L-tryptophan-producing ability may be imparted by enhancing expression of a gene which encodes tryptophan synthase in the tryptophan operon (*trpBA*). The tryptophan synthase consists of α and β subunits, which are encoded by *trpA* and *trpB,* respectively. In addition, L-tryptophan-producing ability may be improved by enhancing expression of the isocitrate lyase-malate synthase operon (WO2005/103275).

### L-Proline-producing bacteria

Examples of L-proline-producing bacteria and parent strains for deriving L-proline-producing bacteria include, but are not limited to, strains belonging to the genus *Escherichia,* such as *E. coli* 702ilvA (VKPM B-8012) which is deficient in the *ilvA* gene and is able to produce L-proline (EP 1172433).

The bacterium used in the present invention may be improved by enhancing the expression of one or more genes involved in L-proline biosynthesis. Examples of such genes preferred for L-proline producing bacteria include the *proB* gene encoding glutamate kinase of which feedback inhibition by L-proline is desensitized (DE Patent 3127361). In addition, the bacterium used in the present invention may be improved by enhancing the expression of one or more genes encoding proteins which act to secrete L-amino acids from bacterial cell. Such genes are exemplified by *b2682* and *b2683* genes (*ygaZH* genes) (EP 1239041 A2) .

Examples of bacteria belonging to the genus *Escherichia,* which have an activity to produce L-proline, include the following *E. coli* strains: NRRL B-12403 and NRRL B-12404 (GB Patent 2075056), VKPM B-8012 (Russian patent application 2000124295), plasmid mutants described in DE Patent 3127361, plasmid mutants described by Bloom F.R. et al (The 15th Miami winter symposium, 1983, p. 34), and so forth.

### L-Arginine-producing bacteria

Examples of L-arginine-producing bacteria and parent strains for deriving L-arginine-producing bacteria include, but are not limited to, strains belonging to the genus *Escherichia,* such as *E. coli* strain 237 (VKPM B-7925) (U.S. Patent Published Application No. 2002/058315 A1) and its derivative strains harboring mutant N-acetylglutamate synthase (Russian Patent Application No. 2001112869), *E. coli* strain 382 (VKPM B-7926) (EP 1170358A1), and an arginine-producing strain into which the *argA* gene encoding N-acetylglutamate synthetase is introduced (EP 1170361 A1).

Examples of L-arginine-producing bacteria and parent strains for deriving L-arginine-producing bacteria also include strains in which expression of one or more genes encoding an L-arginine biosynthetic enzyme are enhanced. Examples of such genes include N-acetylglutamyl phosphate reductase gene (*argC*), ornithine acetyl transferase gene (*argJ*), N-acetylglutamate kinase gene (*argB*), acetylornithine transaminase gene (*argD*), ornithine carbamoyl transferase gene (*argF*), argininosuccinic acid synthetase gene (*argG*), argininosuccinic acid lyase gene (*argH*), and carbamoyl phosphate synthetase gene (*carAB*).

### L-Valine-producing bacteria

Example of L-valine-producing bacteria and parent strains for deriving L-valine-producing bacteria include, but are not limited to, strains which have been modified to overexpress the *ilvGMEDA* operon (U.S. Patent No. 5,998,178). It is desirable to remove the region which includes the *ilvGMEDA* operon, which is required for attenuation, so that expression of the operon is not attenuated by the L-valine that is produced. Furthermore, the *ilvA* gene in the operon is desirably disrupted so that threonine deaminase activity is decreased.

Examples of L-valine-producing bacteria and parent strains for deriving L-valine-producing bacteria also include mutants having a mutation of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 has been deposited at the Russian National Collection of Industrial Microorganisms (VKPM) (1 Dorozhny proezd., 1 Moscow 117545, Russia) on June 24, 1988 under an accession number VKPM B-4411.

Furthermore, mutants requiring lipoic acid for growth and/or lacking H⁺-ATPase can also be used as parent strains (WO96/06926).

### L-Isoleucine-producing bacteria

Examples of L-isoleucine producing bacteria and parent strains for deriving L-isoleucine producing bacteria include, but are not limited to, mutants having resistance to 6-dimethylaminopurine (JP 5-304969 A), mutants having resistance to an isoleucine analogue such as thiaisoleucine and isoleucine hydroxamate, and mutants additionally having resistance to DL-ethionine and/or arginine hydroxamate (JP 5-130882 A). In addition, recombinant strains transformed with genes encoding proteins involved in L-isoleucine biosynthesis, such as threonine deaminase and acetohydroxate synthase, can also be used as parent strains (JP 2-458 A, FR 0356739, and U.S. Patent No. 5,998,178).

When the aforementioned L-amino acid-producing bacteria are bred by gene recombination, the genes to be used are not limited to genes having the genetic information mentioned above or genes having known sequences, but also include genes having conservative mutations, such as homologues or artificially modified genes, can also be used so long as the functions of the encoded proteins are not degraded. That is, they may be genes encoding a known amino acid sequence containing one or more substitutions, deletions, insertions, additions or the like of one or several amino acid residues at one or several positions.

Although the number of the "several" amino acid residues referred to herein may differ depending on the position in the three-dimensional structure or the types of amino acid residues of the protein, specifically, it may be preferably 1 to 20, more preferably 1 to 10, still more preferably 1 to 5. The conservative substitution is a mutation wherein substitution takes place mutually among Phe, Trp, and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Typical examples of a conservative mutation are conservative substitutions, and substitutions considered conservative substitutions include, specifically, substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned amino acid substitutions, deletions, insertions, additions, inversions or the like may be a result of a naturally-occurring mutation or a variation due to an individual difference or difference of species of a microorganism from which the genes are derived (mutant or variant). Such genes can be obtained by, for example, modifying a known nucleotide sequence of a gene by site-specific mutagenesis so that the amino acid residues at the specific sites of the encoded protein include substitutions, deletions, insertions, or additions of amino acid residues.

Furthermore, such genes having conservative mutation(s) as mentioned above may encode a protein having a homology of 80% or more, preferably 90% or more, more preferably 95% or more, particularly preferably 97% or more, to the entire encoded amino acid sequence and having a function equivalent to that of the wild-type protein.

Moreover, codons in the gene sequences may be replaced with other codons which are easily used in the host into which the genes are introduced.

The genes having conservative mutation(s) may be obtained by methods usually used in mutagenesis treatments such as treatments with mutagenesis agents.

Furthermore, the genes may be a DNA which can hybridize with a complementary sequence of a known gene sequence or a probe which can be prepared from the complementary sequence under stringent conditions and encodes a protein having a function equivalent to that of the known gene product. The "stringent conditions" referred to here are conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, particularly preferably not less than 97% homologous, hybridize to each other, and DNAs less homologous than the above do not hybridize to each other, or conditions of washing once, preferably 2 or 3 times, at a salt concentration and temperature corresponding to washing typical of Southern hybridization, i.e., 1 x SSC, 0.1% SDS at 60°C, preferably 0.1 x SSC, 0.1% SDS at 60°C, more preferably 0.1 x SSC, 0.1% SDS at 68°C.

As the probe, a part of the sequence which is complementary to the gene can also be used. Such a probe can be prepared by PCR using oligonucleotides prepared on the basis of the known gene sequence as primers and a DNA fragment containing the nucleotide sequences as a template. For example, when a DNA fragment having a length of about 300 bp is used as the probe, the washing conditions of hybridization may be 50°C, 2 x SSC and 0.1% SDS.

### <3> Method for producing L-amino acid

In the method for producing an L-amino acid of the present invention, a bacterium belonging to the family *Enterobacteriaceae* and having an L-amino acid-producing ability is cultured in a medium containing crude glycerol from biodiesel fuel production as the carbon source to produce and accumulate the L-amino acid in the culture, and the L-amino acid is collected from the culture.

The crude glycerol from biodiesel fuel production may be used at any concentration so long as it is a concentration suitable for production of the L-amino acid. When crude glycerol from biodiesel fuel production is used as the sole carbon source in the medium, about 0.1 to 50% w/v, more preferably about 0.5 to 40% w/v, particularly preferably about 1 to 30% w/v % is present in the medium. Crude glycerol from biodiesel fuel production can also be used in combination with other carbon sources such as glucose, fructose, sucrose, blackstrap molasses, and starch hydrolysate. In this case, although crude glycerol from biodiesel fuel production and other carbon sources may be mixed at an arbitrary ratio, it is desirable that the ratio of glycerol in the carbon source should be 10% by weight or more, more preferably 50% by weight or more, still more preferably 70 % by weight or more. Preferred as the other carbon sources are saccharides such as glucose, fructose, sucrose, lactose, galactose, blackstrap molasses, starch hydrolysate, and a sugar solution obtained by hydrolysis of biomass, alcohols such as ethanol, and organic acids such as fumaric acid, citric acid, and succinic acid. Among these, glucose is preferred. Particularly preferred is a mixture containing crude glycerol and glucose at a weight ratio of 50:50 to 90:10.

Although an initial concentration of glycerol at the time of start of the culture is as described above, glycerol may be supplemented with consumption of glycerol during the culture.

In the present invention, a medium is a medium to which crude glycerol from biodiesel fuel production is added. Crude glycerol may be added to the medium so that glycerol is present at a concentration within the aforementioned range in terms of the amount of glycerol depending on purity of the glycerol.

Furthermore, both glycerol and crude glycerol from biodiesel fuel production may be added to the medium.

As the medium to be used, media conventionally used in the production of L-amino acids by fermentation using microorganisms can be used. That is, conventional media containing, besides a carbon source, a nitrogen source, inorganic ions, and optionally other organic components as required may be used. As the nitrogen source, inorganic ammonium salts such as ammonium sulfate, ammonium chloride, and ammonium phosphate, organic nitrogen such as soybean hydrolysate, ammonia gas, aqueous ammonia, and so forth may be used. As for organic trace nutrient sources, it is desirable that the medium contains required substances such as vitamin B₁, L-homoserine, and yeast extract, or the like in appropriate amounts. Other than the above, potassium phosphate, magnesium sulfate, iron ions, manganese ions and so forth are added in small amounts, as required. In addition, the medium used in the present invention may be either a natural medium or synthetic medium, so long as it contains a carbon source, a nitrogen source, inorganic ions, and other organic trace components as required.

The culture is preferably performed for 1 to 7 days under aerobic conditions. The culture temperature is preferably 24 to 45°C, and the pH during the culture is preferably 5 to 9. To adjust the pH, inorganic or organic acidic or alkaline substances, ammonia gas, and so forth can be used. Collection of the L-amino acid from the culture medium can usually be attained by a combination of known methods such as an ion exchange resin method and precipitation method. When the L-amino acid accumulates in the cells, the cells can be disrupted with, for example, supersonic waves or the like, and the L-amino acid can be collected by the ion exchange resin method or the like from the supernatant obtained by removing the cells from the cell-disrupted suspension by centrifugation.

### Examples

Hereafter, the present invention will be still more specifically explained with reference to examples. In the examples, glycerol of reagent special grade (Nakalai Tesque) was used as reagent glycerol, and crude glycerols produced in biodiesel fuel production process (GLYREX, Nowit DCA-F and R Glycerin) were used as crude glycerol. As for glycerol purity of these crude glycerols, the crude glycerol GLYREX had a purity of 86% by weight, the crude glycerol Nowit DCA-F had a purity of 79% by weight, and the crude glycerol R Glycerin had a purity of 78% by weight.

GLYREX was obtained as crude glycerol produced by FOX PETROLI (S.P.A. Sede legale e uffici, via Senigallia 29, 61100 Pesaro, Italy), and marketed by SVG (SVG ITALIA, SrL Via A. Majani, 2, 40122 Bologna (BO), Italy) as an animal feed additive. Nowit DCA-F is marketed by Nordische Oelwerke Walther Carrouxy GmbH & Co KG, Postfach 930247 Industriestrasse 61-65, 21107 Hamburg, Germany. Glycerin R is crude glycerol marketed by Inter-Harz GmbH, Postfach 1411 Rostock-Koppel 17, 25314 Elmshom, 25365 K1. Offenseth-Sparrieshoop, Germany.

### Example 1: Growth of a wild-type strain in minimal medium

The *Escherichia coli* MG1655 strain was cultured at 37°C for 16 hours on LB agar medium (10 g/L of tryptone, 5 g/L of yeast extract, 10 g/L of NaCl, 15 g/L of agar), and the cells were scraped with a loop and suspended in a 0.9% NaCl solution. This suspension was inoculated into 5 ml of M9 medium (12.8 g/L of Na₂HPO₄·7H₂O, 0.6 g/L of K₂HPO₄, 0.5 g/L of NaCl, 1 g/L of NH₄Cl, 2 mM MgSO₄, 0.1 mM CaCl₂) containing 0.4% (w/v) of either glucose, the reagent glycerol, or crude glycerol, as a carbon source, and the cells were cultured at 37°C for 24 hours in a test tube.

This culture medium was diluted, and inoculated on the LB agar medium, and the cells were cultured at 37°C for 16 hours. In order to accurately measure the degree of growth, the grown colonies were counted as a count of viable cells. Averages of the results of the culture performed in test tubes in duplicate are shown in Table 1.

**Table 1**

| Carbon source | Viable cell count (per ml) |
|---|---|
| Glucose | 1.0 x 10⁵ |
| Reagent glycerol | 1.2 x 10⁵ |
| Crude glycerol GLYREX | 6.6 x 10⁶ |

With the reagent glycerol, the growth was equivalent to, or more than, that observed with glucose, and with the crude glycerol, unexpected good growth, i.e., 50 times or more growth as compared to that observed with glucose, was observed.

### Example 2: L-Threonine production culture

The *Escherichia coli* VKPM B-5318 strain, which is an L-threonine-producing bacterium, was cultured at 37°C for 24 hours on LB agar medium (10 g/L of tryptone, 5 g/L of yeast extract, 10 g/L of NaCl, 15g/L of agar) containing 20 mg/L of streptomycin sulfate. The cells on the agar medium were scraped, inoculated into 20 mL of an L-threonine production medium containing 20 mg/L of streptomycin sulfate in a 500 ml-volume Sakaguchi flask, and cultured at a temperature of 40°C for 24 hours. The main culture was performed in media independently using glucose, the reagent glycerol, and the crude glycerol, or using glucose and the reagent glycerol at a ratio of 1:1, or glucose and the crude glycerol at a ratio of 1:1, as a carbon source. The total amount of the carbon source was 40 g/L for all the cases.

### (Composition of L-threonine production medium)

| (Group A) | |
|---|---|
| Carbon source | 40 g/L |
| MgSO₄·7H₂O | 1 g/L |

| (Group B) | |
|---|---|
| Yeast extract | 2 g/L |
| FeSO₄·7H₂O | 10 mg/L |
| MnSO₄·4H₂O | 10 mg/L |
| KH₂PO₄ | 1 g/L |
| (NH₄)₂SO₄ | 16 g/L |

| (Group C) | |
|---|---|
| Calcium carbonate | 30 g/L |

The components of Groups A and B were subjected to autoclave sterilization at 115°C for 10 minutes, the component of Group C was subjected to hot air sterilization at 180°C for 3 hours, and after the components of the three groups were cooled to room temperature, they were mixed and used.

After completion of the culture, consumption of the added saccharide and glycerol was confirmed with BF-5 (Oji Scientific Instruments), and the degree of the growth was measured in terms of turbidity (OD) at 600 nm. The amount of L-threonine was measured by liquid chromatography. Averages of the results of the culture performed in flasks in duplicate are shown in Table 2.

Under the main culture conditions, when the amount of L-threonine was low when glucose was used as the carbon source, a marked improvement of the L-threonine amount was observed when the reagent glycerol was added independently or as a mixture. Furthermore, when crude glycerol was independently used, the increase in the L-threonine amount was higher than that observed when the reagent glycerol was independently used.

**Table 2**

| Carbon source | OD | Thr (g/l) |
|---|---|---|
| Glucose 40 g/l | 9.6 | 3.8 |
| Glucose 20 g/l + reagent glycerol 20 g/l | 11.6 | 12.3 |
| Reagent glycerol 40 g/l | 10.1 | 9.9 |
| Glucose 20 g/l + crude glycerol GLYREX 20 g/l | 11.2 | 12.5 |
| Crude glycerol GLYREX 40 g/l | 10.4 | 12.0 |

### Example 3: L-lysine production culture

The *Escherichia coli* WC196ΔcadAΔldc/pCABD2 strain, described in International Patent Publication WO2006/078039 (this strain is called "WC196LC/pCABD2"), was used as an L-lysine-producing bacterium. The *Escherichia coli* WC196LC/pCABD2 was cultured at 37°C for 24 hours on LB agar medium (10 g/L of tryptone, 5 g/L of yeast extract, 10 g/L of NaCl, 15g/L of agar) containing 20 mg/L of streptomycin sulfate. The cells on the agar medium were scraped, inoculated into 20 mL of an L-lysine production medium containing 20 mg/L of streptomycin sulfate in a 500 ml-volume Sakaguchi flask, and cultured at a temperature of 37°C for 48 hours. The main culture was performed in media independently using glucose, the reagent glycerol, and the crude glycerol, or using glucose and the reagent glycerol at a ratio of 1:1, or glucose and the crude glycerol at a ratio of 1:1, as a carbon source. The total amount of the carbon source was 40 g/L for all the cases.

### (Composition of L-lysine production medium)

| (Group A) | |
|---|---|
| Carbon source | 40 g/L |

| (Group B) | |
|---|---|
| Yeast extract | 2 g/L |
| FeSO₄·7H₂O | 10 mg/L |
| MnSO₄·4H₂O | 10 mg/L |
| KH₂PO₄ | 1 g/L |
| (NH₄)₂SO₄ | 24 g/L |

| (Group C) | |
|---|---|
| Calcium carbonate | 30 g/L |

The components of Groups A and B were subjected to autoclave sterilization at 115°C for 10 minutes, the component of Group C was subjected to hot air sterilization at 180°C for 3 hours, and after the components of the three groups were cooled to room temperature, they were mixed and used.

After completion of the culture, consumption of the added saccharide and glycerol was confirmed with BF-5 (Oji Scientific Instruments), and the degree of the growth was measured in terms of turbidity (OD) at 600 nm. The amount of L-lysine was measured with a Biotech Analyzer AS210 (Sakura Seiki). Averages of the results of the culture performed in flasks in duplicate are shown in Table 3.

Compared with the amount of L-lysine observed by using glucose as the carbon source, the amount of L-lysine markedly decreased when the reagent glycerol was added as a mixture or independently. However, when crude glycerol was used as a mixture or independently, the amount of L-lysine increased as compared to when the reagent glycerol was used, and the L-lysine amount was equivalent to the L-lysine amount observed when glucose is the carbon source.

**Table 3**

| Carbon source | OD | Lys (g/l) |
|---|---|---|
| Glucose 40 g/l | 8.6 | 14.9 |
| Glucose 20 g/l + reagent glycerol 20 g/l | 10.4 | 13.3 |
| Reagent glycerol 40 g/l | 10.0 | 13.4 |
| Glucose 20 g/l + crude glycerol GLYREX 20 g/l | 10.7 | 14.3 |
| Crude glycerol GLYREX 40 g/l | 9.9 | 14.5 |

### Example 4: L-Lysine production culture with various crude glycerols

The *Escherichia coli* WC196LC/pCABD2 strain, which is an L-lysine-producing bacterium, was cultured at 37°C for 24 hours on LB agar medium (10 g/L of tryptone, 5 g/L of yeast extract, 10 g/L of NaCl, 15g/L of agar) containing 20 mg/L of streptomycin sulfate. The cells on the agar medium were scraped, inoculated into 20 mL of an L-lysine production medium containing 20 mg/L of streptomycin sulfate in a 500 ml-volume Sakaguchi flask, and cultured at a temperature of 37°C for 48 hours. The main culture was performed in media independently using glucose, the reagent glycerol, and the crude glycerols, GLYREX, Nowit DCA-F, or R Glycerin, as the carbon source. The total amount of the carbon source was 40 g/L for all the cases.

After completion of the culture, consumption of the added saccharide and glycerol was confirmed with BF-5 (Oji Scientific Instruments), and the degree of the growth was measured in terms of turbidity (OD) at 600 nm. The amount of L-lysine was measured with a Biotech Analyzer AS210 (Sakura Seiki). Averages of the results of the culture performed in flasks in duplicate are shown in Table 4.

Compared with the amount of L-lysine observed by using the regent glucose as the carbon source, the amount of L-lysine increased with all the crude glycerols, GLYREX, Nowit DCA-F, and R Glycerin.

**Table 4**

| Carbon source | OD | Lys (g/l) |
|---|---|---|
| Glucose 40 g/l | 9.7 | 14.9 |
| Crude glycerol GLYREX 40 g/l | 9.8 | 16.6 |
| Crude glycerol Nowit DCA-F 40 g/l | 10.0 | 15.9 |
| Crude glycerol R Glycerin 40 g/l | 9.9 | 16.4 |

### Example 5: Construction of an L-glutamic acid-producing Pantoea ananatis strain

A plasmid RSFPPG was constructed, which corresponds to the plasmid RSFCPG carrying the citrate synthase gene (*gltA*), phosphoenolpyruvate carboxylase gene (ppc), and glutamate dehydrogenase gene (*gdhA*) derived from *Escherichia coli* (refer to European Patent Laid-open No. 1233068) in which the *gltA* gene was replaced with the methyl citrate synthase gene (*prpC*) of *Escherichia coli* (International Patent Publication WO2006/051660).

Primer 1 (SEQ ID NO: 1) and Primer 2 (SEQ ID NO: 2) were designed to amplify the part of the *gltA* gene of RSFCPG other than the ORF. By using these primers and RSFCPG as the template, PCR was performed to obtain a fragment of about 14.9 kb. Furthermore, as for the methyl citrate synthase gene (*prpC*) derived from *Escherichia coli,* PCR was performed by using Primer 3 (SEQ ID NO: 3), Primer 4 (SEQ ID NO: 4) and the *Escherichia coli* W3110 strain chromosomal DNA as the template to obtain a fragment of about 1.2 kb. Both the PCR products were treated with *Bgl*II and *Kpn*I, and ligated, and the ligation product was used to transform the *Escherichia coli* JM109 strain. All the colonies that appeared were collected, and plasmids were extracted from them as a mixture. This plasmid mixture was used to transform the *Escherichia coli* ME8330 strain, which is a citrate synthase (CS) deficient strain, and the cells were applied to the M9 minimal medium (12.8 g/L of Na₂HPO₄·7H₂O, 0.6 g/L of K₂HPO₄, 0.5 g/L of NaCl, 1 g/L of NH₄Cl, 2 mM MgSO₄, 0.1 mM CaCl₂) containing 50 mg/L of uracil and 5 mg/L of thiamine HCl. A plasmid was extracted from the strains that appeared, as RSFPPG. The plasmid RSFPPG was introduced into the *Pantoea ananatis* NP106 strain, which is an L-glutamic acid-producing bacterium, to construct an L-glutamic acid-producing bacterium, NP106/RSFPPG (this strain is called "NA1 strain").

The NP106 strain was obtained as follows. The *Pantoea ananatis* AJ13601 strain exemplified above was cultured overnight at 34°C in the LBGM9 liquid medium with shaking, and the medium was diluted so that 100 to 200 colonies emerged per plate, and were then applied to an LBGM9 plate containing 12.5 mg/L of tetracycline. The colonies that appeared were replicated to an LBGM9 plate containing 12.5 mg/L of tetracycline and 25 mg/L of chloramphenicol, and the strain which demonstrated sensitivity to chloramphenicol was selected to obtain a strain in which pSTVCB was eliminated, which was designated G106S. Furthermore, the G106S strain was cultured overnight at 34°C in the LBGM9 liquid medium with shaking, and the medium was diluted so that 100 to 200 colonies emerge per plate, and were then applied to an LBGM9 plate containing no drug. The colonies that appeared were replicated to an LBGM9 plate containing 12.5 mg/L of tetracycline and an LBGM9 plate containing no drug, and a strain which became tetracycline sensitive was selected to obtain a strain in which RSFCPG was eliminated, which was designated NP106. The NP106 strain obtained as described above corresponds to the AJ13601 strain not harboring both plasmids RSFCPG and pSTVCB.

### Example 6: L-Glutamic acid production culture

The *Pantoea ananatis* NA1 strain, which is an L-glutamic acid-producing bacterium, was cultured at 34°C for 24 hours on the LBM9 agar medium (10 g/L of tryptone, 5 g/L of yeast extract, 10 g/L of NaCl, 12.8 g/L of Na₂HPO₄·7H₂O, 0.6 g/L of K₂HPO₄, 0.5 g/L of NaCl, 1 g/L of NH₄Cl, 5 g/l of glucose, 15 g/L of agar) containing 12.5 mg/L of tetracycline hydrochloride. The cells on the agar medium were scraped, inoculated into 5 mL of an L-glutamic acid production medium containing 12.5 mg/L of tetracycline hydrochloride in a test tube, and cultured at a temperature of 34°C for 24 hours. The main culture was performed in media independently using sucrose, glucose, the reagent glycerol, and the crude glycerol as a carbon source. The total amount of the carbon source was 30 g/L for all the cases.

### (Composition of L-glutamic acid production medium)

| (Group A) | |
|---|---|
| Carbon source | 30 g/L |
| MgSO₄·7H₂O | 0.5 g/L |

| (Group B) | |
|---|---|
| (NH₄)₂SO₄ | 20 g/L |
| KH₂PO₄ | 2 g/L |
| FeSO₄·7H₂O | 20 mg/L |
| MnSO₄·4H₂O | 20 mg/L |
| Yeast extract | 2 g/L |
| Calcium pantothenate | 18 mg/L |

| (Group C) | |
|---|---|
| Calcium carbonate | 20 g/L |

The components of Groups A and B were subjected to autoclave sterilization at 115°C for 10 minutes, the component of Group C was subjected to hot air sterilization at 180°C for 3 hours, and after the components of the three groups were cooled to room temperature, they were mixed and used.

After completion of the culture, the degree of the growth was measured in terms of turbidity (OD) at 600 nm, and consumption of the added glucose and glycerol was confirmed with BF-5 (Oji Scientific Instruments). The amounts of sucrose and L-glutamic acid were measured with a Biotech Analyzer AS 210 (Sakura Seiki). Averages of the results of the culture performed in test tubes in duplicate are shown in Table 5.

Compared with the amount of L-glutamic acid observed by using glucose as the carbon source, the amount of L-glutamic acid was markedly increased when the reagent glycerol was used. Furthermore, when the crude glycerol GLYREX was used, a markedly larger L-glutamic acid amount was observed as compared to when glucose or regent glycerol was used, and a larger L-glutamic acid amount could be obtained as compared to when sucrose was used.

**Table 5**

| Carbon source | OD | Glu (g/l) |
|---|---|---|
| Sucrose 40 g/l | 12.4 | 16.8 |
| Glucose 40 g/l | 13.6 | 14.1 |
| Regent glycerol 40 g/l | 14.0 | 14.9 |
| Crude glycerol GLYREX 40 g/l | 13.6 | 17.7 |

### Example 7: Component analysis of crude glycerol

Component analysis of the crude glycerols, GLYREX, Nowit DCA-F and R Glycerin, was performed. The measurement methods are as follows. Glycerol and methanol were measured by gas chromatography. Total nitrogen was measured by the Kjeldahl method, and the ether soluble fraction was measured by the Soxhlet extraction method. Formic acid and acetic acid were measured by high performance liquid chromatography, and chloride ions and sulfate ions were measured by ion chromatography. Sodium, potassium, and copper were measured by atomic absorption spectrophotometry, and phosphorus, iron, calcium, magnesium, manganese and zinc were measured by ICP (Inductively Coupled Plasma) emission spectrometry. The results of the measurements are shown as contents per 100 g (g) in Table 6.

**Table 6**

| Measurement item | GLYREX | Nowit DCA-F | R Glycerin |
|---|---|---|---|
| Glycerol | 85.9 | 78.5 | 78.2 |
| Total nitrogen | <0.01 | <0.01 | <0.01 |
| Ether soluble fraction | 0.1 | 0.3 | <0.1 |
| Na | 0.16 | 2.09 | 2.11 |
| K | 2.45 | 0.0062 | 0.144 |
| Cl⁻ | 2.33 | 2.62 | 3.38 |
| SO₄²⁻ | <0.05 | 0.06 | <0.05 |
| Methanol | 0.0054 | 0.11 | 0.0015 |
| Formic acid | 0.02 | 0.01 | <0.01 |
| Acetic acid | 0.03 | 0.02 | 0.03 |
| P | 0.0085 | 0.0787 | 0.0219 |
| Mg | 0.001 | 0.0003 | 0.0003 |
| Fe | 0.0036 | 0.00043 | 0.00054 |
| Ca | 0.0032 | 0.0011 | <0.001 |
| Mn | 0.00003 | 0.00001 | 0.00001 |
| Cu | 0.00002 | 0.00008 | <0.00001 |
| Zn | 0.00077 | <0.00001 | <0.00001 |
| Na + K + Cl⁻ + SO₄²⁻* | 4.94 | 4.7762 | 5.63 |
| Mg + Fe + Ca * | 0.0078 | 0.00183 | 0.00084 |
| Mn + Cu + Zn * | 0.00082 | 0.00009 | 0.00001 |

| | | | |
|---|---|---|---|
| * A value of 0 was used for the results below the measurement limits for calculation. | | | |

### [Explanation of Sequence Listing]

SEQ ID NO: 1: Primer for amplifying part of *gltA* gene other than ORF
SEQ ID NO: 2: Primer for amplifying part of *gltA* gene other than ORF
SEQ ID NO: 3: Primer for amplifying *prpC* gene
SEQ ID NO: 4: Primer for amplifying *prpC* gene

### Industrial Applicability

According to the present invention, L-amino acids can be produced at a low cost by using the inexpensive carbon source crude glycerol from biodiesel fuel production.

## Claims

1. A method for producing an L-amino acid, which comprises culturing a bacterium belonging to the family Enterobacteriaceae and having an L-amino acid-producing ability in a medium containing crude glycerol obtainable in biodiesel fuel production as the carbon source to produce and accumulate the L-amino acid in the culture, and collecting the L-amino acid from the culture.

2. The method according to claim 1, wherein the initial concentration of the glycerol in the medium is 1 to 30% w/v.

3. The method according to any one of claims 1 to 2, wherein the bacterium belongs to the genus Escherichia.

4. The method according to any one of claims 1 to 2, wherein the bacterium belongs to the genus Pantoea.

5. The method according to claim 3, wherein the bacterium is Escherichia coli.

6. The method according to any one of claims 1 to 5, wherein the L-amino acid consists of one or more kinds of L-amino acids selected from the group consisting of L-threonine, L-glutamic acid and L-lysine.

7. The method according to claim 6, wherein the L-amino acid is L-threonine, and activity or activities of one or more kinds of enzymes selected from the group consisting of aspartate semialdehyde dehydrogenase, and aspartokinase I, homoserine kinase, aspartate aminotransferase and threonine synthase encoded by the thr operon are enhanced in the bacterium.

8. The method according to claim 6, wherein the L-amino acid is L-lysine, and activity or activities of one or more kinds of enzymes selected from the group consisting of dihydrodipicolinate reductase, diaminopimelate decarboxylase, diaminopimelate dehydrogenase, phosphoenolpyruvate carboxylase, aspartate aminotransferase, diaminopimelate epimerase, aspartate semialdehyde dehydrogenase, tetrahydrodipicolinate succinylase and succinyl diaminopimelate deacylase are enhanced, and/or activity of lysine decarboxylase is attenuated in the bacterium.

9. The method according to claim 6, wherein the L-amino acid is L-glutamic acid, and the activity or activities of one or more kinds of enzymes selected from the group consisting of glutamate dehydrogenase, citrate synthase, phosphoenolpyruvate carboxylase and methyl citrate synthase are enhanced, and/or activity of α-ketoglutarate dehydrogenase is attenuated in the bacterium.

10. The method according to any one of claims 1 to 5, wherein the L-amino acid is L-tryptophan, and the activity or activities of one or more kinds of enzymes selected from the group consisting of phosphoglycerate dehydrogenase, 3-deoxy-D-arabinoheptulonate-7-phosphate synthase, 3-dehydroquinate synthase, shikimate dehydratase, shikimate kinase, 5-enolpyruvate shikimate 3-phosphate synthase, chorismate synthase, prephenate dehydratase and chorismate mutase are enhanced in the bacterium.

## Patentansprüche

1. Verfahren zum Produzieren einer L-Aminosäure, umfassend das Kultivieren eines Bakteriums, welches zur Familie Enterobacteriaceae gehört und eine Fähigkeit zur Produktion von L-Aminosäure aufweist, in einem Medium, das Rohglycerin (crude glycerol), erhältlich in der Biodieselkraftstoffproduktion, als Kohlenstoffquelle enthält, um die L-Aminosäure in der Kultur zu produzieren und zu akkumulieren, und das Gewinnen der L-Aminosäure aus der Kultur.

2. Verfahren gemäß Anspruch 1, wobei die anfängliche Konzentration des Glycerins in dem Medium 1 bis 30% w/v beträgt.

3. Verfahren gemäß irgendeinem der Ansprüche 1 bis 2, wobei das Bakterium zur Gattung Escherichia gehört.

4. Verfahren gemäß irgendeinem der Ansprüche 1 bis 2, wobei das Bakterium zur Gattung Pantoea gehört.

5. Verfahren gemäß Anspruch 3, wobei das Bakterium Escherichia coli ist.

6. Verfahren gemäß irgendeinem der Ansprüche 1 bis 5, wobei die L-Aminosäure aus einer oder mehreren Arten von L-Aminosäuren besteht, ausgewählt aus der Gruppe, bestehend aus L-Threonin, L-Glutaminsäure und L-Lysin.

7. Verfahren gemäß Anspruch 6, wobei die L-Aminosäure L-Threonin ist und die Aktivität oder Aktivitäten einer oder mehrerer Arten von Enzymen, ausgewählt aus der Gruppe, bestehend aus Aspartatsemialdehyddehydrogenase und Aspartokinase I, Homoserinkinase, Aspartataminotransferase und Threoninsynthase, codiert durch das thr-Operon, in dem Bakterium verstärkt ist/sind.

8. Verfahren gemäß Anspruch 6, wobei die L-Aminosäure L-Lysin ist und die Aktivität oder Aktivitäten einer oder mehrerer Arten von Enzymen, ausgewählt aus der Gruppe, bestehend aus Dihydrodipicolinatreduktase, Diaminopimelatdecarboxylase, Diaminopimelatdehydrogenase, Phosphoenolpyruvatcarboxylase, Aspartataminotransferase, Diaminopimelatepimerase, Aspartatsemialdehyddehydrogenase, Tetrahydrodipicolinatsuccinylase und Succinyldiaminopimelatdeacylase, in dem Bakterium verstärkt ist/sind und/oder die Aktivität von Lysindecarboxylase abgeschwächt ist.

9. Verfahren gemäß Anspruch 6, wobei die L-Aminosäure L-Glutaminsäure ist und die Aktivität oder Aktivitäten einer oder mehrerer Arten von Enzymen, ausgewählt aus der Gruppe, bestehend aus Glutamatdehydrogenase, Citratsynthase, Phosphoenolpyruvatcarboxylase und Methylcitratsynthase, in dem Bakterium verstärkt ist/sind und/oder die Aktivität von α-Ketoglutaratdehydrogenase abgeschwächt ist.

10. Verfahren gemäß irgendeinem der Ansprüche 1 bis 5, wobei die L-Aminosäure L-Tryptophan ist und die Aktivität oder Aktivitäten einer oder mehrerer Arten von Enzymen, ausgewählt aus der Gruppe, bestehend aus Phosphoglyceratdehydrogenase, 3-Desoxy-D-arabinoheptulonat-7-phosphatsynthase, 3-Dehydrochinatsynthase, Shikimatedehydratase, Shikimatekinase, 5-Enolpyruvatshikimate-3-phosphatsynthase, Chorismatsynthase, Prephenatdehydratase und Chorismatmutase, in dem Bakterium verstärkt ist/sind.

## Revendications

1. Procédé pour produire un L-acide aminé, qui comprend la mise en culture d'une bactérie appartenant à la famille Enterobacteriaceae et présentant une capacité de production de L-acide aminé dans un milieu contenant du glycérol brut pouvant être obtenu par la production de carburant biodiesel en tant que source de carbone pour produire et accumuler le L-acide aminé dans la culture, et la collecte du L-acide aminé depuis la culture.

2. Procédé selon la revendication 1, dans lequel la concentration initiale du glycérol dans le milieu est de 1 à 30 % en poids/volume.

3. Procédé selon l'une quelconque des revendications 1 à 2, dans lequel la bactérie appartient au genre Escherichia.

4. Procédé selon l'une quelconque des revendications 1 à 2, dans lequel la bactérie appartient au genre Pantoea.

5. Procédé selon la revendication 3, dans lequel la bactérie est la Escherichia coli.

6. Procédé selon l'une quelconque des revendications 1 à 5, dans lequel le L-acide aminé consiste en une ou plusieurs sortes de L-acides aminés sélectionnés à partir du groupe constitué par la L-thréonine, le L-acide glutamique et la L-lysine.

7. Procédé selon la revendication 6, dans lequel le L-acide aminé est la L-thréonine, et l'activité ou les activités d'une ou plusieurs sortes d'enzymes sélectionnées à partir du groupe constitué par l'aspartate semi-aldéhyde déshydrogénase, et l'aspartokinase I, l'homosérine kinase, l'aspartate aminotransférase et la thréonine synthase codée par l'opéron de thr sont accrues dans la bactérie.

8. Procédé selon la revendication 6, dans lequel le L-acide aminé est la L-lysine, et l'activité ou les activités d'une ou plusieurs sortes d'enzymes sélectionnées à partir du groupe consistant en la dihydrodipicolinate réductase, la diaminopimélate décarboxylase, la diaminopimélate déshydrogénase, la phosphoénolpyruvate carboxylase, l'aspartate aminotransférase, la diaminopimélate épimérase, l'aspartate semi-aldéhyde déshydrogénase, la tétrahydrodipicolinate succinylase et la succinyl diaminopimélate désacylase sont accrues, et/ou l'activité de la lysine décarboxylase est atténuée dans la bactérie.

9. Procédé selon la revendication 6, dans lequel le L-acide aminé est le L-acide glutamique, et l'activité ou les activités d'une ou plusieurs sortes d'enzymes sélectionnées à partir du groupe consistant en la glutamate déshydrogénase, la citrate synthase, la phosphoénolpyruvate carboxylase et la méthyle citrate synthase sont accrues, et/ou l'activité de la α-cétoglutarate déshydrogénase est atténuée dans la bactérie.

10. Procédé selon l'une quelconque des revendications 1 à 5, dans lequel le L-acide aminé est le L-tryptophane, et l'activité ou les activités d'une ou plusieurs sortes d'enzymes sélectionnées à partir du groupe consistant en le phosphoglycérate déshydrogénase, la 3-désoxy-D-arabinoheptulonate-7-phosphate synthase, la 3-déshydroquinate synthase, la shikimate déshydratase, la shikimate kinase, la 5-énolpyruvate shikimate 3-phosphate synthase, la chorismate synthase, la préphénate déshydratase et la chorismate mutase sont accrues dans la bactérie.
